# EUROPEAN PATENT SPECIFICATION

(11) **EP 1 600 441 B1**
(45) Date of publication and mention of the grant of the patent: **30.12.2009**
(21) Application number: 05106264.4
(22) Date of filing: 05.08.2002
(51) Int. Cl.: C07D 211/90, A61K 31/44, A61P 9/12

(54) **Crystal form of lercanidipine hydrochloride for use as an antihypertensive agent**
Kristallform von Lercanipidin-Hydrochlorid zur Verwendung als antihypertensives Mittel
Forme cristalline du chlorhydrate de lercanidipine utile comme agent anti-hypertensif

(30) Priority: 06.08.2001 IT MI20011726
(43) Date of publication of application: 30.11.2005
(62) Divisional of application: 02762428.7
(73) Proprietor: Recordati Ireland Limited, Ringaskiddy County Cork (IE)
(72) Inventor: Bonifacio, Fausto, 04100 Latina (IT); Campana, Francesco, 00040 Rocca Priora (IT); De Iasi, Gianluca, 04011 Aprilia (IT); Leonardi, Amedeo, 20154 Milano (IT)
(74) Representative: HOFFMANN EITLE

(56) References cited:
- WO-A-96/35668
- WO-A-97/03669
- US-A- 5 767 136
- MCCLELLAN K J ET AL: "LERCANIDIPINE A REVIEW OF ITS USE IN HYPERTENSION" DRUGS, ADIS INTERNATIONAL LTD, AT, vol. 5, no. 60, November 2000 (2000-11), pages 1123-1140, XP008001723 ISSN: 0012-6667

## Description

### FIELD OF THE INVENTION

The invention is directed to the crystalline form (I) of lercanidipine hydrochloride, for use in an antihypertensive treatment method and to processes for the preparation of said form.

### BACKGROUND OF THE INVENTION

Lercanidipine (methyl 1,1,N-trimethyl-N-(3,3-diphenylpropyl)-2-aminoethyl 1,4-dihydro-2,6-dimethyl-4-(3-nitrophenyl)pyridine-3,5-dicarboxylate) is a highly lipophilic dihydropyridine calcium antagonist with long duration of action and high vascular selectivity. Its mechanism of antihypertensive activity is attributed to a direct relaxant effect on vascular smooth muscle, which lowers total peripheral resistance. The recommended starting dose of lercanidipine as monotherapy is 10 mg daily by oral route, with a drug titration as necessary to 20 mg daily. Lercanidipine is rapidly absorbed following oral administration with peak plasma levels occurring 2-3 hours following dosing. Elimination is essentially via the hepatic route.

By virtue of its high lipophilicity and high membrane coefficient, lercanidipine combines a short plasma half life with a long duration of action. In fact, the preferential distribution of the drug into membranes of smooth muscle cells results in membrane-controlled pharmacokinetics characterized by a prolonged pharmacological effect. In comparison to other calcium antagonists, lercanidipine is characterized by gradual onset and long-lasting duration of action despite decreasing plasma levels. *In vitro* studies show that isolated rat aorta response to high K⁺ are attenuated by lercanidipine for 6 hours even after the drug has been removed from the environment of the aortic tissue.

Lercanidipine is commercially available from Recordati S.p.A. (Milan, Italy) and has been described along with methods for making it and resolving it into individual enantiomers in U.S. Patents 4,705,797; 5,767,136; 4,968,832; 5,912,351; and 5,696,139.

A process for preparing lercanidipine described in U.S. Patent No. 4,705,797 involves the following scheme: 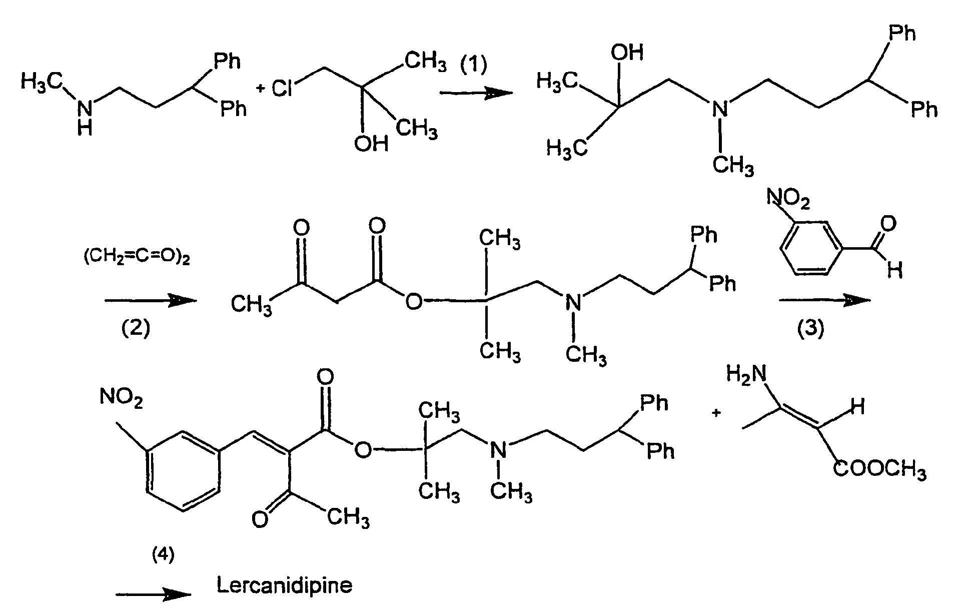 (1): xylene at reflux; (2): toluene, 85°C; (3) HCl +CHCl₃; 0°C; (4) HO-CH(CH₃)₂ at reflux Crude lercanidipine is an oily residue that must be purified by flash chromatography using chloroform, containing increasing amounts of acetone, as the eluant. The solvent is then evaporated to dryness and remaining residue is dissolved in methanol adding a small excess of hydrochloric acid in ethanol. After evaporation of the solvent, the hemi-hydrated hydrochloride salt is prepared by treatment with diluted hydrochloric acid in the presence of sodium chloride, this product is the amorphous form

A major disadvantage of the process of preparing lercanidipine, as it is described in U.S. Patent No. 4,705,797, is that the disclosed cyclization reaction generates several by-products, which results in a lower yield for the desired product. Moreover, the purification and isolation of lercanidipine from the reaction mixture is quite complex, since it requires numerous treatments with different solvents. Finally, the purification and isolation steps are difficult to perform on an industrial scale because of the necessity of purifying the product by column chromatography. U.S. Patent 5,912,351 describes a simpler process for the preparation of lercanidipine hydrochloride. It involves reaction of 1,4-dihydro-2,6-dimethyl-5-methoxycarbonyl-4-(3-nitrophenyl)-pyridine-3-carboxylic acid with thionyl chloride in dichloromethane and dimethylformamide at a temperature between -4 and +1°C and subsequent esterification of the obtained acid chloride with 2-N-dimethyl-N-(3,3-diphenylpropyl)-1-amino-2-propyl alcohol at a temperature between -10 and 0°C. The process yields lercanidipine hydrochloride in an anhydrous non-hygroscopic crystalline form, and avoids the formation of unwanted by-products and the subsequent purification on chromatography columns.

However, the isolation of lercanidipine hydrochloride in crystalline form is again quite complex. After evaporating the solvent from the reaction mixture and dissolving the residue thus obtained in ethyl acetate, the solution is washed first with brine, then washed further five times with a 10% solution of sodium carbonate, five times with 1 N hydrochloric acid, and eventually once again with brine.

Therefore, there is a need in the art for a process for the preparation of lercanidipine hydrochloride in crystalline form which avoids one or more of the disadvantages of the currently used processes.

In addition, it was observed that lercanidipine, as produced by the second-described process above, displayed batch-to-batch variability despite careful process control and even observation of the melting point believed to be characteristic of the solid product obtained by the process of Example 3 of USP 5,767,136 of 186-188°C. This variability was manifest in seemingly unpredictably appearing (and disappearing) differences in one or more of product appearance (e.g., color), melting point and solubility. This raised issues as to whether assurances of purity and/or reproducibility can be made (e.g., to regulatory authorities) that the product is always the same.

WO 96/35668 discloses a process for the preparation of lercanidipine hydrochloride involving reaction of an acid halide of 2,6-dimethyl-5-methoxycarbonyl-4-(3-nitrophenyl)-1,4-dihydropyridine-3-carboxylic acid with 2,N-dimethyl-N-(3,3-diphenylpropyl)-1-amino-2-propanol in an aprotic solvent. The product is obtained by industrially applicable crystallisation techniques.

Further research by the present inventors revealed batch-to-batch differences In bioavailability in animals, and differences in crystal size, in the course of researching the causes of the variability problem, the inventors surprisingly discovered novel lercanidipine hydrochloride polymorphs. They also discovered more suitable processes for the preparation and isolation of crystalline lercanidipine hydrochloride products from the reaction mixture. It was surprisingly determined that lercanidipine hydrochloride shows polymorphic features and crystallizes into different crystalline forms depending on the process followed and on the solvents used. Furthermore, the isolation of each of individual crystalline polymorphs has become possible, thus decreasing the possibility of batch to batch variability of lercanidipine, which the present inventors determined was due to mixtures of different solid forms being present by the same batch and to such mixtures of different composition having melting points within the same narrow range as the individual forms. As a result, more reproducible batches of lercanidipine more suitable for large scale manufacture and quality control were needed.

### SUMMARY OF THE INVENTION

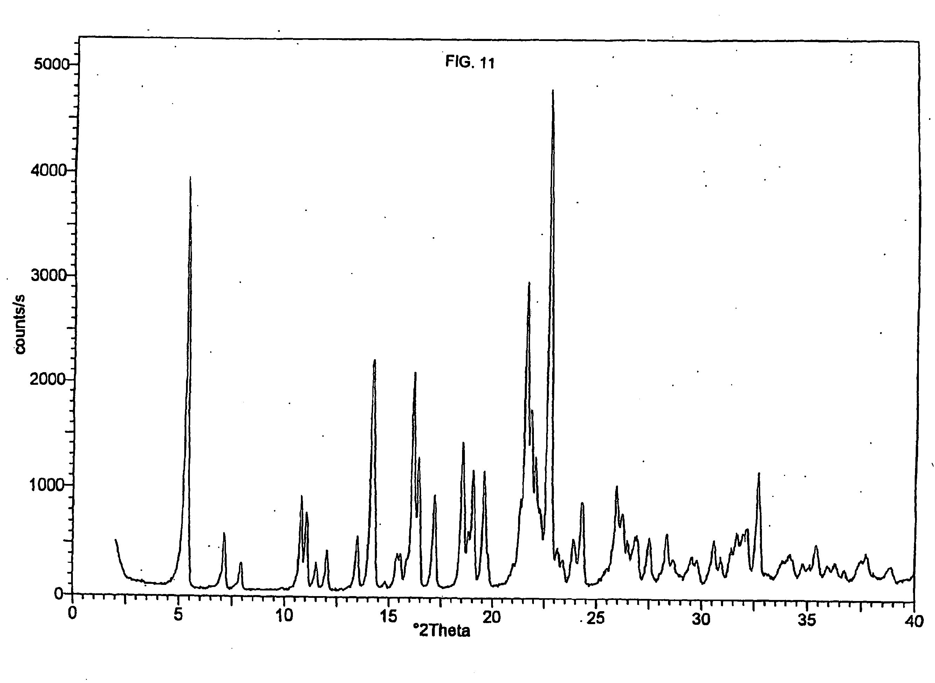

The present invention relates to lercanidipine hydrochloride crystalline Form (I) having an X-ray powder diffraction pattern as shown in Figure 11, for use in an antihypertensive treatment method.

The distances D(X), relative intensity ratios (I/I₀) ratios, and 2 θ angles of significant peaks are:

| D (X) | Relative intensity (l/lo) | 2 θ angle |
|---|---|---|
| 16.3 | 83 | 5.4 |
| 6.2 | 47 | 14.2 |
| 4.78 | 29 | 18.6 |
| 4.10 | 63 | 21.7 |
| 4.06 | 36 | 21.9 |
| 3.90 | 100 | 22.8 |

The lercanidipine hydrochloride crystalline Form (I) is characterized by a Raman spectrum with the peaks as specified in Table 8.

The lercanidipine hydrochloride crystalline Form (I) has a melting point of about 197-201°C, when said melting point is determined as DSC

Also provided are methods of syntheses for preparing lercanidipine hydrochloride crystalline Form (I) from a lercanidipine hydrochloride crude form.

### BRIEF DESCRIPTION OF THE DRAWINGS

**Figure 1** is a graph of DSC analysis carried out on crystalline Form (I), according to the working conditions described in Example 3. The ordinate indicates heat flow in mW and the abscissa temperature in °C.
**Figure 2** is a graph of DSC analysis carried out on crystalline Form (II) (Reference), according to the working conditions described in Example 3. The ordinate indicates heat flow in mW and the abscissa temperature in °C.
**Figure 3** is a graph of the results of the thermogravimetric tests carried out on Form (I) and Form (II) (Reference), respectively, as described in Example 4. The abscissa indicates temperature in °C and the ordinate indicates percent mass variation.
**Figure 4** is a graph of solubility at 25°C of Forms (I) and (II) (Reference) in ethanol at increasing water concentrations. The experiments are described in Example 6. The ordinate indicates % solubility expressed as w/w and the abscissa % by weight of water in ethanol.
**Figure 5** is a graph of solubility at 40°C of Forms (I) and (II) (Reference) in ethanol at increasing water concentrations. The tests are described in Example 6. The ordinate indicates % solubility expressed as w/w and the abscissa % by weight of water in ethanol.

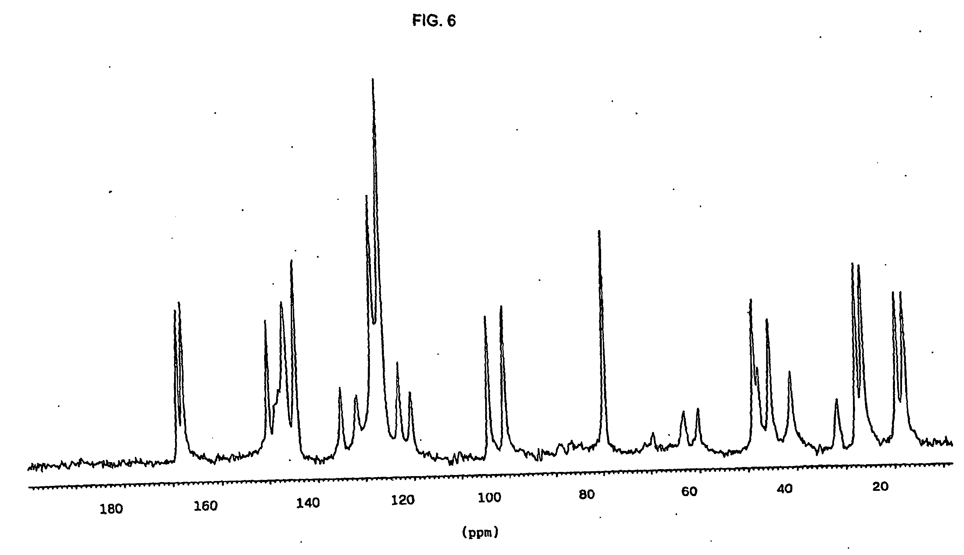

**Figure 6** shows ¹³C NMR spectra in solid phase of crystalline Form (I). The signals and attributes of the corresponding carbon atoms can be found in Table 4.

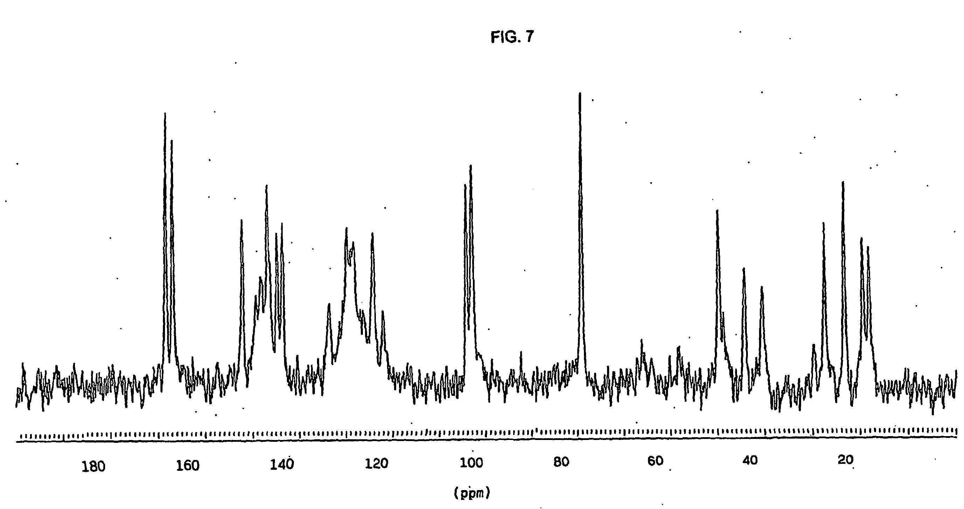

**Figure 7** shows ¹³C NMR spectra in solid phase of crystalline Form (II) (Reference). The signals and attributes of the corresponding carbon atoms can be found in Table 5.

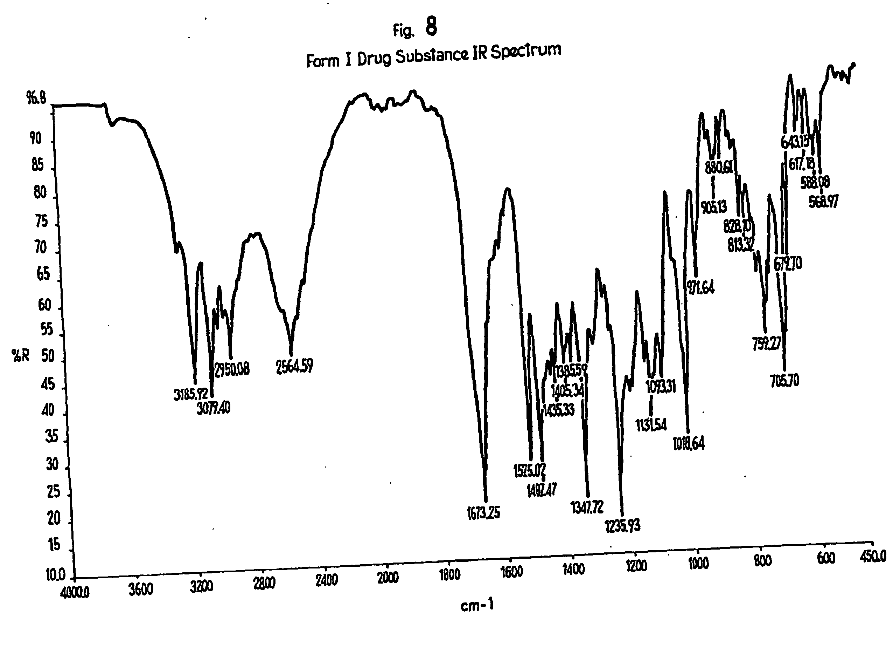

**Figure 8** shows IR spectra of Form (I). The signal and corresponding attributes can be found in Table 6.

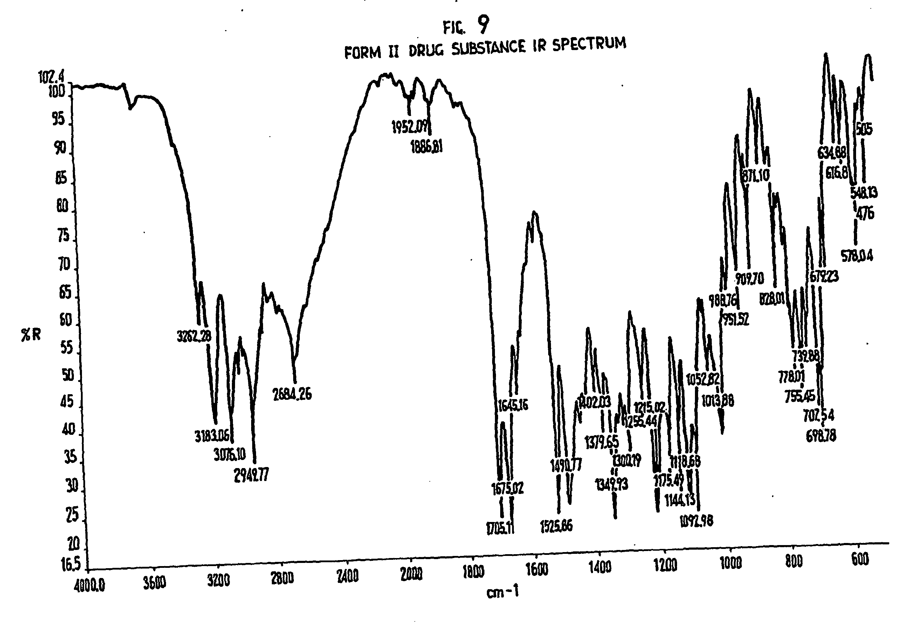

**Figure 9** shows IR spectra of Form (II) (Reference). The signal and corresponding attributes can be found in Table 7.

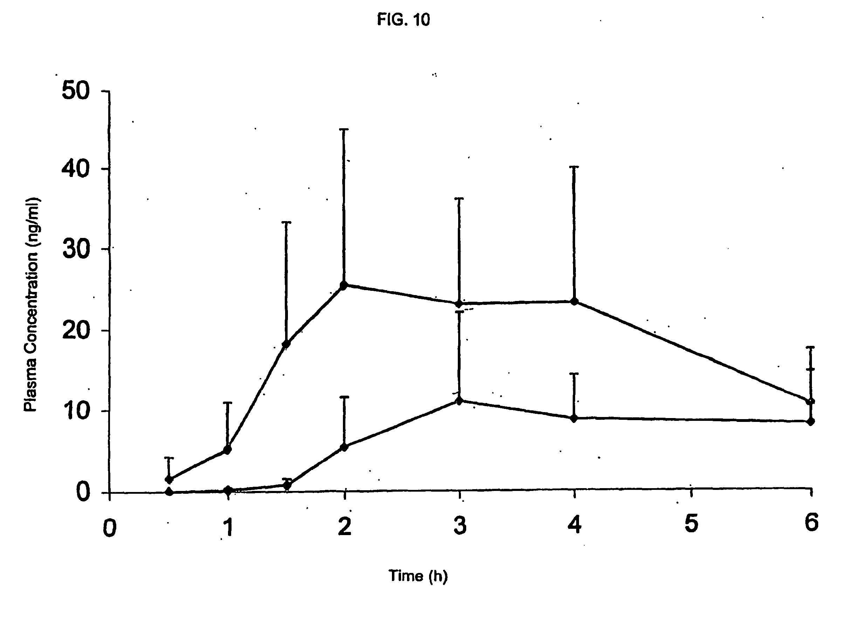

**Figure 10** represents percent average concentration of lercanidipine hydrochloride in dog plasma after administration of crystalline Form (I) and of crystalline Form (II) (Reference) in an amount of 3 mg/kg, in the form of a hard gelatin capsule. The ordinate indicates the mean value of concentration in plasma and the abscissa indicates time (in minutes).
**Figures 11** and **12** show X-ray diffraction spectra at wavelength Kα of crystalline Forms (I) and (II) (Reference), respectively. The distances (d) in X, the (I/lo) ratios and values of 2θ angles of the most significant peaks can be found in Tables 10 and 11 below. The ordinate indicates the number of counts/sec and the abscissa shows the values of 2θ angles.
**Figures 13** and **14** are plots of percent mass change as a function of time in hygroscopicity tests carried out on Forms (I) and (II) (Reference) of lercanidipine hydrochloride, respectively. The ordinate on the left indicates percent mass changes and the ordinate on the right percent relative humidity; the abscissa indicates time in minutes. The protocol for the hygroscopicity tests are described in Example 14.

### DETAILED DESCRIPTION OF THE INVENTION

The crystalline form (I) of lercanidipine hydrochloride can be obtained from lercanidipine hydrochloride crude Forms (A) and (B).Isolated Form (I) may be reproducibly obtained from the (A) and (B) intermediates by varying the crystallization conditions as described below. Forms (I) may also be obtained using other starting materials. Forms (I) may be obtained using, for example, crude lercanidipine.HCl Form (C) as starting material, as described herein.

Lercanidipine hydrochloride crystalline Forms (I) exhibits good stability. Form (I) is characterized by a paler yellow color, smaller crystal size, higher solubility in aqueous media (all compared to Form (II)), and a melting point (DSC peak) within the rage of about 197°C to about 201°C, more specifically, about 198.7°C, and the X-ray diffraction pattern set forth, supra.

Reference Form (II) is characterized by a more pronounced yellow color, larger crystal size, slightly lower solubility in aqueous media (all compared to Form (I)), and a melting point (DSC peak) within the range of about 207-211°C, more specifically about 209.3°C.

Reference Form (II) exhibited higher bioavailability in the dog, and was also non equivalent to form I in man, showing a higher plasma concentration (AUCo-t) and a delayed time of maximum concentration (tmax), compared to Form (I).

Previously known methods for producing crystalline lercanidipine hydrochloride were inconsistent in producing lercanidipine hydrochloride with predictable physical and chemical characteristics. Hence, the previously known methods had the undesirable property of producing lercanidipine hydrochloride that varied, e.g., in physico-chemical properties, from batch to batch, even among batches produced by the same process and under the same conditions. The present inventors have discovered that the source of inconsistency exhibited by the previously known methods for producing lercanidipine hydrochloride is the presence of varying and unpredictable amounts of crystalline lercanidipine hydrochloride Form (II). In contrast to previously known methods for producing lercanidipine hydrochloride, the invention provides crystalline Form for use in an antihypertensive treatment method (I) of a purity and uniformity that has not been obtained with previously achieved crystalline forms of lercanidipine hydrochloride.

The purity and uniformity of Form (I) allows for increased ease in production of lercanidipine dosage forms, due to, e.g., more precisely defined physico-chemical characteristics, such as, for example, increased uniformity of particle size prior to micronization and more reproducible solubility. Form (I) also provides dosage forms with more precisely defined pharmacological characteristics, e.g., bioavailability, compared to previously achieved dosage forms that varied from batch-to-batch in their physico-chemical characteristics.

In a human study in man, where the plasma levels of lercanidipine were assessed after administration of a single dose of either lercanidipine hydrochloride Form (I) or (II) (Reference), Form (I) had a shorter time in obtaining the maximum concentration in plasma, relative to Form (II). Hence, Form (I) is more suited for immediate release formulations and dosage forms.

As used herein, the term "crude form" refers to precipitated solid forms comprising crystals of a compound that have not been washed and/or recrystallized to remove impurities (including but not limited to solvent) that may be present. In the present specification, the crude forms are referred to as Forms (A) and (B) of lercanidipine hydrochloride.

As used herein, the term "crystalline form" refers to crystals of a compound that have been washed and recrystallized to remove impurities. In the present invention, the term crystalline forms refers to Forms (I) and (II) (Reference) of lercanidipine hydrochloride. These crystalline forms have an HPLC purity ≥ 99.5 % and residual solvents content of < 3000 ppm.

As used herein, the term "polymorphism" refers to a property of a compound to crystallize in two or more forms with distinct structures. The different crystalline forms can be detected directly by crystallographic techniques or indirectly by assessment of differences in physical and/or chemical properties associated with each particular polymorph.

As used herein, a "subject in need of treatment" is a mammalian (e.g., human) subject suffering from or at risk of developing the particular condition to be treated, e.g., essential hypertension, secondary hypertension, isolated systolic hypertension , coronary heart disease (e.g., chronic stable angina, myocardial infarction), congestive heart failure. A subject in need of treatment for arterial hypertension may be identified using methods well known in the art such as, for example, by direct measurement of blood pressure using, for example, a manual sphygmomanometer, automatic/electronic devices or ambulatory blood pressure monitoring.

The present invention contemplates any method that may be used to produce the crude forms of lercanidipine hydrochloride described herein. These forms have different physico-chemical properties, e.g., melting points (which can be determined by DSC analysis), than the crude form of lercanidipine hydrochloride produced by other known methods, e.g., by the method described in U.S. Patent No. 5,912,351; termed Form (C). Form (A) has a melting point of about 150°C to about 152°C (DSC peak), Form (B) has a melting point of about 131°C to about 135°C (DSC peak), and Form (C) has a melting point of about 186°C to about 192°C (DSC peak). Additionally, thermogravimetric studies show that Form (A) comprises 3 - 4 % residual ethyl acetate and Form (B) comprises 0.3-0.7 % residual ethyl acetate, by weight. Comparatively, the residual solvent present in Form (C) has been determined to be 0-0.1 %. Form (A) and (B) are prepared with the processes as described in the parent European Patent Application EP1432683. It has been surprisingly found that each of crude lercanidipine hydrochloride Form (A) and Form (B), when undergoing different purification treatments, result in two different crystalline forms of lercanidipine hydrochloride. Studies indicate that these crystalline forms have different physical and chemical properties. DSC analysis of crystalline Form (I) indicates that it has a melting peak of about 197°C to about 201°C, specifically about 198.7°C. DSC analysis of crystalline Form (II) (Reference) indicates that it has a melting peak of about 207°C to about 211°C, specifically about 209.3°C.

One purification process (y process), that leads to formation of crystalline (Form (I)) comprises the following steps:

### Process for Making Form (I)

a) adding isopropanol to crude lercanidipine hydrochloride (Form (A) or Form (B)) and heating under reflux with stirring to produce a solution (if the solution is not clear, it should be filtered hot);
b) cooling the solution of step a) preferably to a temperature between 30 and 40°C and stirring for a period of time preferably between 12 and 48 hours to produce a solid; and
c) filtering the solid obtained from step b), washing the solid with isopropanol, re-filtering the solid, and drying the solid (e.g., in an oven) at preferably 70°C for a period of time preferably between 12-48 hours.

Crude Form (C) may be also be used as starting material in step a). In such case, however, there is the risk of decreased yield of product because the solution should be filtered hot, resulting in the increased loss of lercanidipine hydrochloride in step a). In step b), crystallization is considered complete when the content of the solution is ≤2% lercanidipine HCl.

Reference Form (II)is prepared with the processes described in EP1432683.

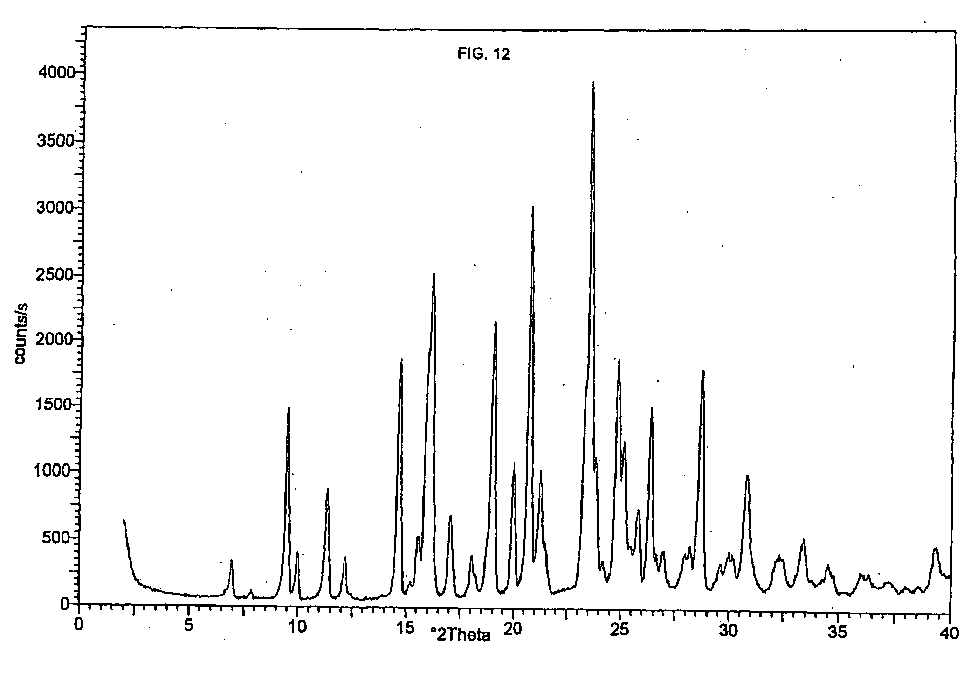

In addition to differences in melting point, the two crystalline forms exhibit differences in x-ray structure, solubility, and bioavailability. Solubility studies show that Form (I) is more soluble than Form (II) (Reference) in water, ethanol, and mixtures thereof (See Tables 2 & 3). Bioavailability studies in humans indicate that Form (II) (Reference) is more bioavailable than Form (I). This study indicates, nevertheless, that Form (I) has a shorter time to maximum concentration attainable and is thus suitable for use in immediate release formulations and dosage forms. Finally, X-ray diffraction studies show that these two forms have different diffraction patterns (see Figures 11 and 12 and Example 11). Form (I) has a smaller crystal and hence smaller particle size before micronization and so is easier and faster to process than Form (II), which presents with larger crystals.

### Pharmaceutical Compositions

The lercanidipine hydrochloride crystalline Form (I) for use in an antihypertensive treatment method according to the present invention may be comprised in a pharmaceutical composition which may include optional additives, such as a pharmaceutically acceptable carrier or diluent, a flavorant, a sweetener, a preservative, a dye, a binder, a suspending agent, a dispersing agent, a colorant, a disintegrant, an excipient, a film forming agent, a lubricant, a plasticizer, an edible oil or any combination of two or more of the foregoing.

The crystalline form (I) undergoes micronization, using any method known in the art. The average size of particle produced by this method is preferably D(50%)2-8 µm, D(90%)<15 µm.

Suitable pharmaceutically acceptable carriers or diluents include: ethanol; water; glycerol; propylene glycol, aloe vera gel; allantoin; glycerin; vitamin A and E oils; mineral oil; PPG2 myristyl propionate; magnesium carbonate; potassium phosphate; vegetable oil; animal oil; and solketal.

Suitable binders include: starch; gelatin; natural sugars, such as glucose, sucrose and lactose; corn sweeteners; natural and synthetic gums, such as acacia, tragacanth, vegetable gum, and sodium alginate; carboxymethylcellulose; hydroxypropylmethylcellulose; polyethylene glycol; povidone; waxes; and the like.

Suitable disintegrants include: starch, e.g., corn starch, methyl cellulose, agar, bentonite, xanthan gum, sodium starch glycolate, crosspovidone and the like.

Suitable lubricants include, but are not limited to, sodium oleate, sodium stearate, sodium stearyl fumarate, magnesium stearate, sodium benzoate, sodium acetate, sodium chloride and the like.

A suitable suspending agent is: bentonite, ethoxylated isostearyl alcohols, polyoxyethylene sorbitol and sorbitan esters, microcrystalline cellulose, aluminum metahydroxide, agar-agar and tragacanth, or mixtures of two or more of these substances, and the like.

Suitable dispersing and suspending agents include: synthetic and natural gums, such as vegetable gum, tragacanth, acacia, alginate, dextran, sodium carboxymethylcellulose, methylcellulose, polyvinyl-pyrrolidone and gelatin.

Suitable film forming agents include: hydroxypropylmethylcellulose, ethylcellulose and polymethacrylates.

Suitable plasticizers include: polyethylene glycols of different molecular weights (e.g., 200-8000 Da) and propylene glycol.

Suitable colorants include:ferric oxide(s), titanium dioxide and natural and synthetic lakes.

Suitable edible oils include: cottonseed oil, sesame oil, coconut oil and peanut oil.

Examples of additional additives include: sorbitol, talc, stearic acid, dicalcium phosphate and polydextrose.

### Unit Dosage Forms

The pharmaceutical composition may be formulated as unit dosage forms, such as tablets, pills, capsules, caplets, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. Unit dosage forms may be used for oral, parenteral, intranasal, sublingual or rectal administration, or for administration by inhalation or insufflation, transdermal patches, and a lyophilized composition. In general, any delivery of active ingredients that results in systemic availability of them can be used.

Preferably the unit dosage form is an oral dosage form, most preferably a solid oral dosage form, therefore the preferred dosage forms are tablets, pills, caplets and capsules. Parenteral preparations (e.g., injectable preparations and preparations for powder jet systems) also are preferred.

Solid unit dosage forms may be prepared by mixing the active agent of the present invention with a pharmaceutically acceptable carrier and any other desired additives as described above. The mixture is typically mixed until a homogeneous mixture of the active agent of the present invention and the carrier and any other desired additives is formed, *i*.*e*., until the active agent is dispersed evenly throughout the composition. In this case, the compositions can be formed as dry or moist granules.

Dosage forms with predetermined amounts of lercanidipine hydrochloride may be formulated starting with compositions with known quantities of lercanidipine hydrochloride using methods well known in the art.

Tablets or pills can be coated or otherwise compounded to form a unit dosage form which has delayed and/or prolonged action, such as time release and sustained release unit dosage forms. For example, the tablet or pill can comprise an inner dosage and an outer dosage component, the latter being in the form of a layer or envelope over the former. The two components can be separated by an enteric layer which serves to resist disintegration in the stomach and permits the inner component to pass intact into the duodenum or to be delayed in release.

Biodegradable polymers for controlling the release of the active agents, include: polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

For liquid dosage forms, the active substances or their physiologically acceptable salts are brought into solution, suspension or emulsion, optionally with the usually employed substances such as solubilizers, emulsifiers or other auxiliaries. Solvents for the active combinations and the corresponding physiologically acceptable salts, can include water, physiological salt solutions or alcohols, e.g. ethanol, propane-diol or glycerol. Additionally, sugar solutions such as glucose or mannitol solutions may be used. A mixture of the various solvents mentioned may further be used.

A transdermal dosage form also is contemplated. Transdermal forms may be a diffusion-driven transdermal system (transdermal patch) using either a fluid reservoir or a drug-in-adhesive matrix system. Other transdermal dosage forms include, topical gels, lotions, ointments, transmucosal systems and devices, and iontophoretic (electrical diffusion) delivery system.

Transdermal dosage forms may be used for timed release and sustained release of the active agent of the present invention.

Pharmaceutical compositions and unit dosage forms for administration parenterally, and in particular by injection, typically include a pharmaceutically acceptable carrier, as described above. A preferred liquid carrier is vegetable oil. Injection may be, for example, intravenous, intrathecal, intramuscular, intraruminal, intratracheal, or subcutaneous.

The active agent also can be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The crystalline form (I) also may be coupled with soluble polymers as targetable drug carriers. Such polymers include : polyvinyl-pyrrolidone, pyran copolymer, polyhydroxypropylmethacryl-amidephenol, polyhydroxy-ethylaspartamidephenol, and polyethyleneoxide, polylysine substituted with palmitoyl residues.

### Administration

The pharmaceutical composition or unit dosage forms comprising lercanidipine hydrochloride crystalline Form I for use in an antihypertensive treatment method according to the present invention may be administered by a variety of routes such as intravenous, intratracheal, subcutaneous, oral, mucosal parenteral, buccal, sublingual, ophthalmic, pulmonary, transmucosal, transdermal, and intramuscular. Unit dosage forms also can be administered in intranasal form via topical use of suitable intranasal vehicles, or via transdermal routes, using those forms of transdermal skin patches known to those of ordinary skill in the art. Oral administration is preferred.

The pharmaceutical composition or unit dosage forms may be administered to an animal, preferably a human being, in need of antihypertensive treatment. The pharmaceutical composition or unit dosage form may be administered according to a dosage and administration regimen defined by routine testing in light of the guidelines given above in order to obtain optimal antihypertensive activity and a decreased in blood pressure while minimizing toxicity or side-effects for a particular patient. However, such fine turning of the therapeutic regimen is routine in light of the guidelines given herein.

The dosage of the composition may vary according to a variety of factors such as underlying disease state, the individual's condition, weight, sex and age and the mode of administration. For oral administration, the pharmaceutical compositions can be provided in the form of scored or unscored solid unit dosage forms.

The pharmaceutical composition preferably comprises (1) lercanidipine hydrochloride crystalline Form (I) and (2) at least one component selected from the group consisting of a pharmaceutically acceptable carrier or diluent, a flavorant, a sweetener, a preservative, a dye, a binder, a suspending agent, a dispersing agent, a colorant, a disintegrant, an excipient, a diluent, a lubricant, a plasticizer, and an edible oil. Preferably, the pharmaceutical composition contains from 0.1 to 400 mg of lercanidipine hydrochloride (1). Preferably, the composition or dosage form comprises 1 to 200 mg lercanidipine hydrochloride (1). More preferably, the composition or dosage form comprises 5 to 40 mg lercanidipine hydrochloride (1).

The pharmaceutical composition or unit dosage form may be administered in a single daily dose, or the total daily dosage may be administered in divided doses. In addition, co-administration or sequential administration of other active agents may be desirable. Polymorph form (I) may be combined with any known drug therapy, preferably for treatment of hypertension. For example, bimodal therapy involving in addition a diuretic, a β-receptor blocker, an ACE inhibitor or an angiotensin II receptor antagonist is contemplated by the present invention (US2003/018035 A1, US2004/0198789)

For combination therapy the compounds may initially be provided as separate dosage forms until an optimum dosage combination and administration regimen is achieved. Therefore, the patient may be titrated to the appropriate dosages for his/her particular hypertensive condition. After the appropriate dosage of each of the compounds is determined to achieve a decrease of the blood pressure without untoward side effects, the patient then may be switched to a single dosage form containing the appropriate dosages of each of the active agents, or may continue with a dual dosage form.

The exact dosage and administration regimen utilizing the combination therapy is selected in accordance with a variety of factors including type, species, age, weight, sex and medical condition of the patient; the severity and etiology of the hypertension to be treated; the route of administration; the renal and hepatic function of the patient; the treatment history of the patient; and the responsiveness of the patient. Optimal precision in achieving concentrations of compounds within the range that yields efficacy without toxicity requires a regimen based on the kinetics of the drug's availability to target sites. This involves a consideration of the absorption, distribution, metabolism, excretion of a drug, and responsiveness of the patient to the dosage regimen. However, such fine tuning of the therapeutic regimen is routine in light of the guidelines given herein.

A pharmaceutical composition for parenteral administration contains not below 0.1%, preferably from about 0.5% to about 30%, by weight of a polymorph (I) based upon the total weight of the pharmaceutical composition.

Generally, transdermal dosage forms contain from about 0.01% to about 100% by weight of the active agent, based upon 100% total weight of the dosage.

Preferably, the composition is administered daily to the patient. Preferably, the pharmaceutical composition has dosage form containing from 0.1 to 400 mg lercanidipine hydrochloride. More preferably, the composition or dosage form comprises 1 to 200 mg lercanidipine hydrochloride. Even more preferably, the composition or dosage form comprises 5 to 40 mg lercanidipine hydrochloride.

### EXAMPLES

The following examples of preparation of lercanidipine hydrochloride crystalline form (I) are now disclosed for illustrative non-limiting purposes, together with the results of DSC analysis and solubility, stability and hygroscopicity tests; the bioavailability tests for the crystalline form (I) are also disclosed and compared with those obtained with form (II) (Reference)

### EXAMPLE 1 Preparation of lercanidipine hydrochloride crystalline Form (I)

In separate representative experiments, 100 g of crude lercanidipine hydrochloride Form (A), (B), or (C) was loaded into a reactor, followed by 400 ml of 2-propanol. The mixture was heated under strong reflux and under stirring, thus obtaining an almost complete dissolution of the crude substance. The mixture was hot filtered to eliminate a slight opalescence and the clear solution kept under stirring was cooled to 40°C. Temperature was then set at 35°C. The whole was kept for 24 hours under stirring at 35°C, then temperature was set at 30°C, and stirring was continued at said temperature for another 24 hours. The solid was filtered at 30°C and washed with 50 ml of 2-propanol, then dried in an oven at 70°C under vacuum for 24 hours. Weight of dry product in each case was (lercanidipine HCl (I)) 90 g (HPLC purity of the product in Form (I) > 99.5%).

### EXAMPLE 1A Preparation of lercanidipine hydrochloride crystalline Form (I)

In separate representative experiments, 100 g of crude lercanidipine hydrochloride Form (A), (B), or (C) was loaded into a reactor, followed by 400 ml of 2-propanol. The mixture was heated under strong reflux and under stirring, thus obtaining an almost complete dissolution of the crude substance. The mixture was hot filtered to eliminate a slight opalescence and the clear solution kept under stirring is slowly cooled to 40°C. Precipitation was then triggered with 100 mg of lercanidipine hydrochloride Form (I) and temperature was set at 35°C, keeping the mixture under stirring. The whole was kept for 24 hours under stirring at 35°C, then temperature was set at 30°C, keeping under stirring at said temperature for another 24 hours. The solid was filtered at 30°C and washed with 50 ml of 2-propanol, then dried in an oven at 70°C under vacuum for 24 hours. Weight of dry product (lercanidipine HCl (I)) was 90 g (HPLC purity of the product in Form (I) > 99.5%).

### EXAMPLE 3 DSC analysis of lercanidipine hydrochloride crystalline Forms (I) and (II) (Reference)

DSC analysis measures changes that occur in a given sample with heating, wherein the changes identify transition phases. Enthalpy variations taking place in a transition phase are calculated on the basis of the area under the curve. The most common transition phases are melting and sublimation. The temperature at which transition starts, onset T, is given by the point in which the curve starts to deviate from the base line (flex point).

DSC of Form (I): 3.8 mg of Form (I) was placed in a golden pan of the apparatus Perkin Elmer DSC7. The heating speed during the test was 10°C/min.

DSC Form (II) (Reference): 4.6 mg of Form (II) was placed in a golden pan of the apparatus Perkin Elmer DSC7. The heating speed during the test was 10°C/min.

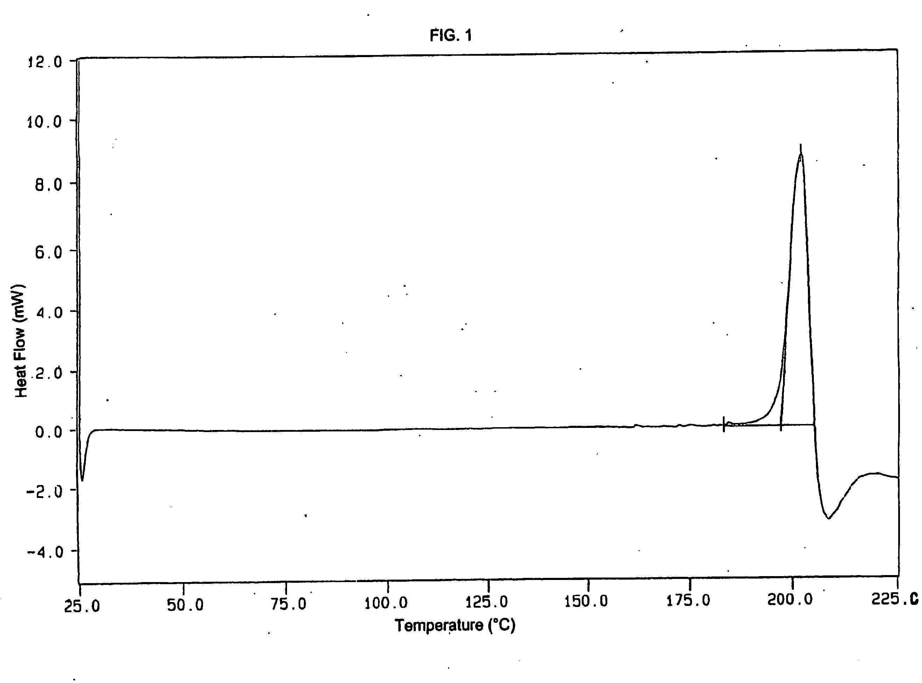

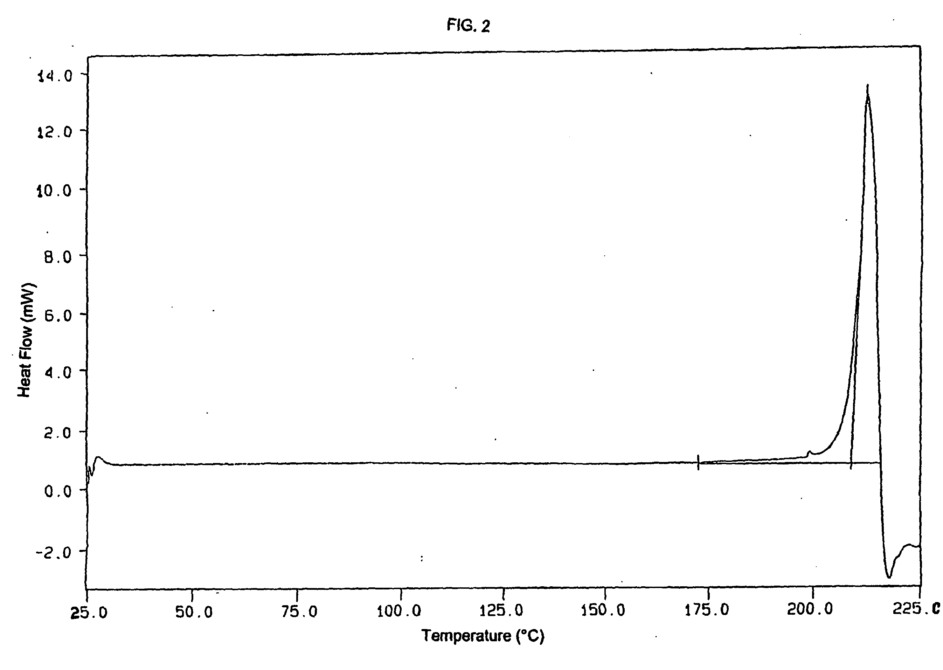

The data are shown in Figures 1 and 2 and the characteristic points of the figures are briefly summarized in the following Table 1.

**Table 1.**

| Compound | Melting T (Tpeak) [°C] | Onset T [°C] |
|---|---|---|
| Form (I) | 198.7 | 179.8 |
| Form (II) (Reference) | 209.3 | 169.0 |

Immediately after melting of Form (I) or (II) (Reference) an exothermic event due to salt decomposition can be observed.

### EXAMPLE 4 Thermogravimetry

A gravimetric analysis associated with an IR analysis was carried out on both crystalline Forms (I) and (II).

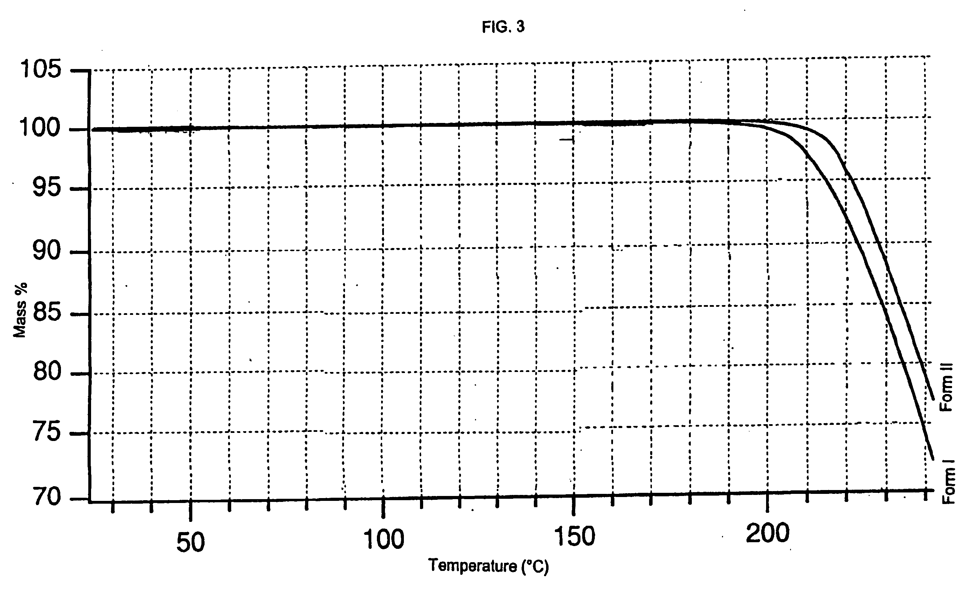

The tests were carried out according to the following working conditions: 2-5 mg of sample was heated in a steel crucible in nitrogen atmosphere, with a heating speed of 10°C/min. The results obtained with crystalline Forms (I) and (II) (Reference) are shown in Figure 3, from which it can be inferred that in both crystalline forms no weight loss can be observed up to their melting point (*i.e.,* until about 190-200°C).

During degradation, which takes places as indicated above after melting, a CO₂ loss can be observed.

### EXAMPLE 5 Hygroscopicity of crystalline Forms (I) and (II) (Reference)

The hygroscopicity of both crystalline Forms (I) and (II) (Reference) was measured with DVS analysis by means of a water absorption analyzer (SURFACE MEASUREMENT SYSTEM, Marion, Buckinghamshire, UK) according to the following working conditions:
10-15 mg of Form (I) and (II) (Reference) respectively were placed in a quartz sample-holder, placed in its turn on a microbalance, and the sample underwent humidity cycles between 0 and 95%, starting from 50% of relative humidity (25°C, relative humidity (RH): 50-95-0-95-0-50% at RH/h:5%).

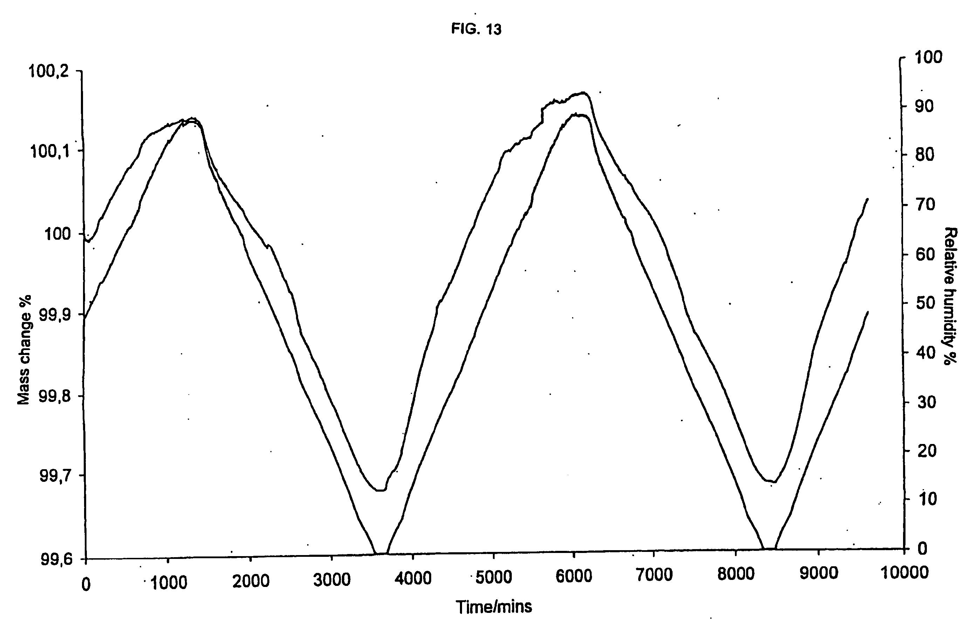

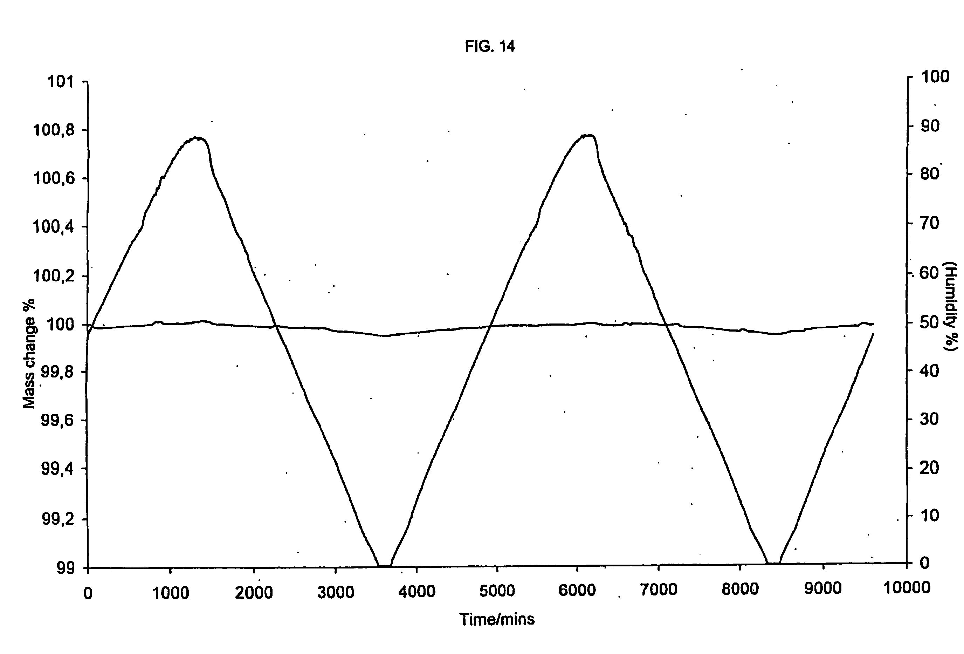

The results of the tests are shown in the diagrams of Figures 13 and 14.

### 14-1 Results obtained with crystalline Form (I)

The exposure of Form (I) to humidity in the DVS analyzer results in a mass change of +0.15% at 95% RH, and of -0.3% at 0% RH, with almost no hysteresis during mass increase and loss. These slight variations are probably due to a reversible surface absorption of water.

### 14-2 Results obtained with crystalline Form (II) (Reference)

The exposure of Form (II) (Reference) to humidity in DVS causes a negligible mass variation (< 0.05%) in the whole RH range tested.

### EXAMPLE 6 Solubility of crystalline Forms (I) and (II) (Reference)

### 6.1 Solubility in water and in ethanol at room temperature

The solubility at 23°C of both crystalline Forms (I) and (II) (Reference) was evaluated by UV-Visible spectroscopy in bi-distilled water (at the pH value spontaneously reached by the system) and in absolute ethanol. The molar absorptivity had been previously determined in acetonitrile. The same molar absorptivity was considered for the determination in water and in ethanol. Solubility in water certainly depends on pH. The residual solid obtained by filtration of the suspension was immediately analyzed with Raman spectroscopy. The results are shown in the following Tables 2 and 3.

**TABLE 2. Solubility in water (about 40 mg/ml as initial condition).**

| Starting material | Time [min] | Solubility [mg/ml] | Residual material |
|---|---|---|---|
| Form (I) | 5/25/45/990 | 0.4/0.5/0.5/0.5 | Form (I) |
| Form (II) (Reference) | 5/25/45/990 | 0.2/0.2/0.3/0.3 | Form (II) |

**TABLE 3. Solubility in ethanol (100 mg/ml as initial condition)**

| Starting material | Time [min] | Solubility [mg/ml] | Residual material |
|---|---|---|---|
| Form (I) | 15/45/120 | 28/27/27 | Form (I) |
| Form (II) (Reference) | 15/45/120 | 11/12/12 | Form (II) |

Form (II) is less soluble than Form (I) in both solvents.

### 6.2 Solubility in mixtures of water-ethanol at 25°C and at 40°C, with increasing water concentrations

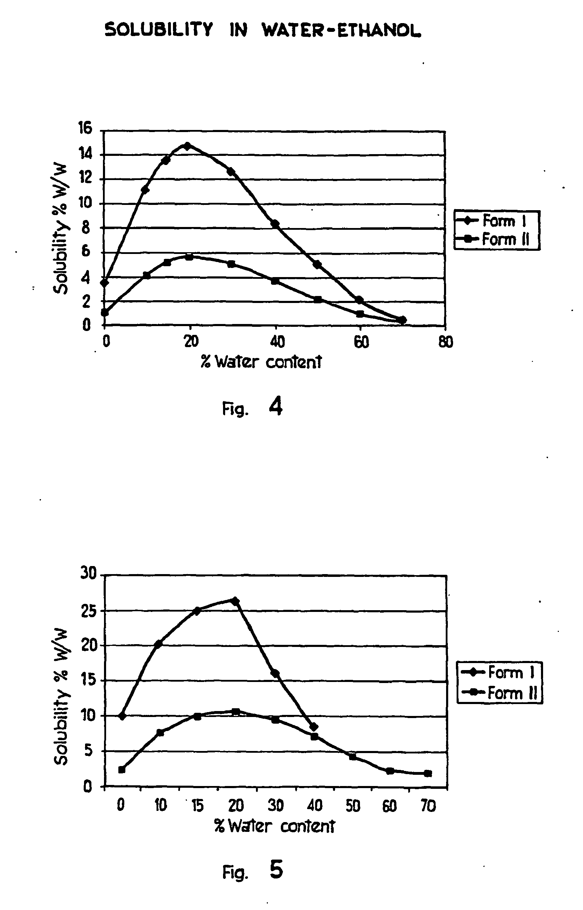

Figures 4 and 5 show solubility in water-ethanol at 25°C and at 40°C of Form (I) and of Form (II) (Reference). The maximum solubility is reached for both forms, at both temperatures, when water concentration is of 20%. Also in this case the solubility of crystalline Form (I) is higher than that of crystalline Form (II).

### EXAMPLE 7 Solid phase ¹³C-NMR studies

The high resolution ¹³C-NMR solid phase spectra were carried out with the Bruker, ASX300 Instrument equipped with a 7 mm Rotor accessory, using several combined techniques:
Magic angle spinning (MAS). About 300 mg of the sample was placed in the rotor spinning at 4.3 kHz around an axis oriented at the magic angle (54° 70') to the magnetic field to overcome the dipolar broadening caused by CSA (Chemical Shift Anisotropy). The experiments were conducted at room temperature.
Dipolar Coupling. Since much of line broadening in ¹³C spectra of organic solids is due to coupling to protons, it was removed by heteronuclear decoupling (decoupling power level was almost 1 Kilowatt).
Cross polarization (CP). Cross polarization allowed carbon magnetization from larger proton magnetization via the dipolar coupling to increase signal intensity.
Total suppression of sidebands (TOSS). TOSS was performed using spin-echoes synchronized with the rotation of the sample to cause phase alteration of the spinning sidebands, resulting in cancellation when successive spectra were added together.

Crystalline Forms (I) and (II) (Reference) show different ¹³C-NMR spectra in solid phase. The signals (chemical shift ) and attribution of the corresponding carbon atoms (as numbered in the formula of lercanidipine hydrochloride shown below) are represented in the following Tables 4 and 5, respectively.

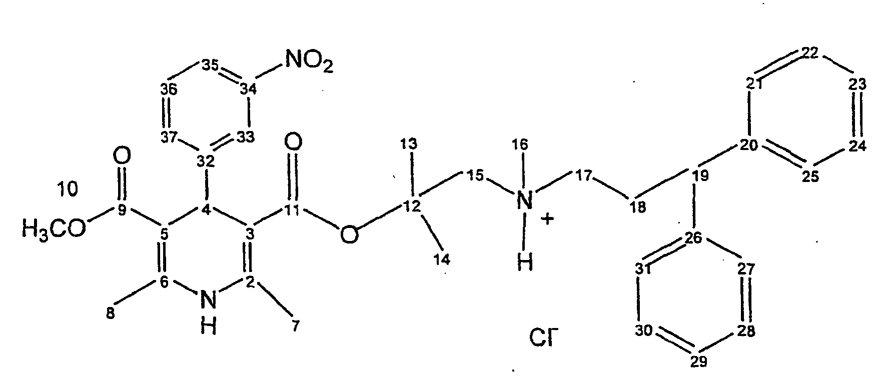

**Table 4. Lercanidipine hydrochloride crystalline Form (I)**

| Chemical shift ( , ppm) | Attribution of carbon atoms |
|---|---|
| 168.7; 167.7 | 9; 11 or 11; 9 |
| 150.1 to 120.4 | 2; 6 and 20 to 37 |
| 104.3; 100.9 | 3; 5 o 5; 3 |
| 79.7 | 12 |
| 63.0; 60.1 (weak) | 15; 17 o r 17;15 |
| 48.6 | 10 |
| 47.7 | 16 |
| 45.4 | 19 |
| 41.1 | 4 |
| 31.6 | 18 |
| 27.7; 26.4 | 13; 14 or 14; 13 |
| 19.6; 18.0 | 7; 8 or 8; 7 |

**Table 5. Lercanidipine hydrochloride crystalline Form (II) (Reference)**

| Chemical shift ( , ppm) | Attribution of carbon atoms |
|---|---|
| 168.1; 166.6 | 9; 11 or 11; 9 |
| 151.9 to 121.9 | 2; 6 and from 20 to 37 |
| 104.0; 102.8 | 3; 5 or 5; 3 |
| 79.0 | 12 |
| 66.0; 58.0 (weak) | 15; 17 or 17;15 |
| 49.7 | 10 |
| 48.8 | 16 |
| 44.3 | 19 |
| 40.5 | 4 |
| 29.8 | 18 |
| 27.6; 23.5 | 13; 14 or 14; 13 |
| 19.6; 18.3 | 7; 8 or 8; 7 |

### EXAMPLE 8 IR Studies

The IR (infrared) spectra were recorded in KBr powder by Diffuse Reflectance Technique using a Perkin Elmer Spectrum-one instrument. IR spectra, whose wavelengths and corresponding attribution are shown in the following Tables 6 and 7, are clearly different for the new Forms (I) and (II).

**Table 6. IR spectrum in KBr powder of lercanidipine hydrochloride Form (I)**

| Wavelength (cm ⁻¹) | Attribution |
|---|---|
| 3186 | NH stretching |
| 3100-2800 | Alkyl and phenyl stretching |
| 2565 | N⁺H stretching |
| 1673 | C=O stretching |
| 1525; 1348 | Asymmetric and symmetric stretching of NO₂ group |
| 1405; 1386 | Bending of geminal methyl groups |
| 785-685 | Out-of-plane bending of 5 and 3 adjacent hydrogens on aromatic rings |

**Table 7. IR spectrum in KBr powder of lercanidipine hydrochloride Form (II) (Reference).**

| Wavelength (cm ⁻¹) | Attribution |
|---|---|
| 3183 | NH stretching |
| 3100-2800 | Alkyl and phenyl stretching |
| 2684 | N⁺H stretching |
| 1705;1675 | C=O stretching |
| 1526; 1350 | Asymmetric and symmetric stretching of NO₂ group |
| 1402; 1380 | Bending of geminal methyl groups |
| 800-680 | Out-of-plane bending of 5 and 3 adjacent hydrogens on aromatic rings |

### EXAMPLE 9: Raman Spectra

A Bruker FT-Raman RFS100 Spectrophotometer was utilized under the following typical conditions: about 10 mg sample (without any previous treatment), 64 scans 2 cm⁻¹ resolution, 100 mW laser power, Ge-detector.

The following Tables 8 and 9 show the most significant peaks of Raman spectra of Form (I) and Form (II) (Reference), respectively.

**Table 8. Raman spectrum of crystalline Form (I)**

| Wave number (cm⁻¹) | Peak intensity * |
|---|---|
| 3054 | M |
| 3040 | M |
| 2981 | M |
| 2941 | M |
| 1675 | S |
| 1646 | M |
| 1583 | M |
| 1489 | M |
| 1349 | Vs |
| 1236 | M |
| 1005 | S |
| 821 | M |
| 174 | M |
| 98 | S |
| 73 | Vs |

| | |
|---|---|
| * M= moderate; S= strong, Vs =very strong | |

**Table 9. Raman spectrum of crystalline Form (II) (Reference)**

| Wave number (cm⁻¹) | Peak intensity* |
|---|---|
| 3074 | M |
| 3064 | M |
| 3055 | M |
| 3048 | M |
| 3030 | M |
| 2973 | M |
| 2940 | M |
| 1675 | S |
| 1647 | S |
| 1630 | M |
| 1584 | M |
| 1489 | M |
| 1351 | Vs |
| 1005 | M |
| 995 | M |
| 103 | Vs |
| 85 | S |

| | |
|---|---|
| *M= moderate; S= strong, Vs =very strong | |

### EXAMPLE 10 Bioavailability of crystalline Forms (I) and (II) (Reference) in man

A study was carried out on 16 healty volunteers to assess the relative bioavailability of lercanidipine hydrochloride Form (I) and Form (II) (Reference). Both Forms (I) and (II) (Reference) were administered in form of a 10 mg tablet prepared exactly in the same way and with the same composition of Zanedip^{R} 10mg, starting from micronized Form (II) (Reference) having the same particle size of Form I (Test-T). Blood samples were taken at 15 points from time 0 to 24 h post-dosing and plasma concentrations of lercanidipine were determined with a stereoselective analytical method HPLC-MS/MS according to the methodology disclosed hereinbelow. Lercanidipine was extracted from dog plasma by means of a liquid-liquid extraction with a mixture of n-hexane and ethyl ether. The dry residue of the organic phase was taken up with a mixture of methanol and water and a liquid-phase chromatographic separation (LC) was carried out; the two enantiomers of lercanidipine were separated on a CHIROBIOTIC V column (Vancomycin) (particle size 5 m, column size 150 x 4.6 mm (ASTEC, NJ, USA)) and were detected with a mass spectrometer (MS/MS) by using an electrospray technique.

The analytical method was validated in a concentration range between 0.1 and 20 ng/ml of plasma for both enantiomers. The method has shown to be specific with an accuracy of 15%. The average concentrations of lercanidipine in the tables represent the sum of both enantiomers. The pharmacokinetic parameters obtained are given in the following table

| | **Form (I) geom. least square mean** | **Form (II) geom. least square mean** | **Point Estimate (T/R)** | **90%C.l.** |
|---|---|---|---|---|
| **AUC** ₀₋ₜ **(ng·h/mL)** | 8.82 | 10.36 | 1.17 | 0.93-1.48 |
| **Cₘₐₓ (ng/mL)** | 3.18 | 3.22 | 1.01 | 0.73-1.42 |
| **tₘₐₓ (h)** | 1.50* | 2.50* | 0.75** | 0.00-1.25 |
| **Cₘₐₓ/AUC** | 0.386^ | 0.329^ | 0.85 | 0.69 -1.02 |

| | | | | |
|---|---|---|---|---|
| * median | | | | |
| ** median difference | | | | |
| ^ least square mean | | | | |

The obtained results indicated that lercanidipine hdyrochloride Form (II) (Reference) was not bioequivalent to Form (I), with Form (II) (Reference) obtaining higher plasma levels, that lercanidipine hydrochloride Form (I) has a tₘₐₓ that is shorter than that of Form (II) (Reference), suggesting its use in immediate release formulations.

### EXAMPLE 11 X-ray diffraction studies

Philips PW 1710 and Philips X pert PW 3040 powder diffractometer (Copper Kα radiation) were used, under the following typical conditions: about 5-70 mg sample (without any previous treatment) with application of a slight pressure to obtain a flat surface. Ambient air atmosphere. 0.02° 2θ stepsize, 2 sec step-1, 2-50 2θ.

The obtained spectra are given in Figures 11 and 12 and the corresponding main peaks are described in the following Tables 10 and 11. The data are clearly different for new isolated Forms (I) and (II) (Reference).

**Table 10. X RD spectrum of lercanidipine hydrochloride Form (I).**

| D (Δ) | Relative intensity (I/lo) | 2 θ angle |
|---|---|---|
| 16.3 | 83 | 5.4 |
| 6.2 | 47 | 14.2 |
| 4.78 | 29 | 18.6 |
| 4.10 | 63 | 21.7 |
| 4.06 | 36 | 21.9 |
| 3.90 | 100 | 22.8 |

**Table 11. X RD spectrum of lercanidipine hydrochloride Form (II).**

| D (Δ) | Relative intensity (I/lo) | 2θ angle |
|---|---|---|
| 9.3 | 35 | 9.5 |
| 6.0 | 45 | 14.7 |
| 5.49 | 65 | 16.1 |
| 4.65 | 52 | 19.1 |
| 4.27 | 74 | 20.8 |
| 3.81 | 41 | 23.4 |
| 3.77 | 100 | 23.6 |
| 3.58 | 44 | 24.8 |
| 3.54 | 29 | 25.2 |

### EXAMPLE 12 Melting point determination of various mixtures of lercanidipine hydrochloride crystalline Forms (I) and (II) (Reference)

The melting points of compositions consisting of known ratios of lercanidipine hydrochloride crystalline Forms (I) and (II) (Reference) were determined in open capillary. Conditions consisted of using a set point of 177°C and introducing the capillary into the instrument (Melting Point Apparatus model 535, Büchi Labortechnik AG, Flawil, Switzerland) at approximately 5°C below the melting point. Results are shown in Table 12.

**Table 12. (Reference) Melting points of compositions consisting of known ratios of lercanidipine hydrochloride crystalline Forms (I) and (II) (Reference). Samples in Series A and Series B were heated at a gradient of 1°C/min and 0.5°C/min, respectively. Results are given in °C.**

| **Sample** | **Pure Form (I)** | **Ratio lercanidipine hydrochloride crystalline Form (I): Form (II)** (Reference) | | | | | **Pure Form (II) (Reference)** |
|---|---|---|---|---|---|---|---|
| | | **9:1** | **7:3** | **1:1** | **3:7** | **1:9** | |
| Series A | 186.8 | 188.0 | 189.5 | 190.0 | 192.2 | 194.2 | 194.3 |
| Series B | 185.9- 186.8 | 184.4- 186.1 | 184.5- 187.0 | 186.7- 187.4 | 186.5- 189.4 | 188.7- 190.5 | 190.6- 192.9 |

U.S. Patent No. 5,767,136 discloses crystalline lercanidipine hydrochloride as having a melting point of 186-188°C. Reference Table 12 shows that this melting point is exhibited by mixtures of Form (I) and Form(II) in which the ratio of Form (I):Form (II) varies between 9:1 to 3:7. Bianchi et al. (Drugs of the Future, 1987, 12:1113-1115) report a melting point of 186-188°C (non DSC) for a lercanidipine product they characterize as "crystals". Hence, the melting point of a preparation of lercanidipine hydrochloride is not sufficient by itself to distinguish the particular form or forms present therein, and many mixtures of different compositions have the same melting point range.

### EXAMPLE 13. Micronization of lercanidipine hydrochloride.

Micronization is carried out by a jet-mill process using a MICRONETTE M300 from the firm NUOVA GUSEO (Villanova sull'Arda -PC- Italy). Parameters are as follows: Injection pressure, 5 Kg/cmq; micronization pressure, 9 Kg/cmq; and cyclone pressure, 2.5 Kg/cmq. Capacity of micronization is 16 Kg/h. Particle size is determined by laser light scattering using a GALAI CIS 1 laser instrument (GALAI, Haifa, Israel). Micronization is performed to obtain an average particle size of D (50%) 2-8 µm and D (90%) < 15 µm.

## Claims

1. Lercanidipine hydrochloride crystalline Form (I) having an X-ray powder diffraction pattern as shown in Figure 11, for use in an antihypertensive treatment method.

2. A method for producing lercanidipine hydrochloride crystalline Form (I), as defined in claim 1, which comprises:
a) adding isopropanol containing a maximum of 5% water (v/v) to a crude lercanidipine hydrochloride Form and heating under reflux and with stirring to produce a clear solution;
b) cooling the solution of step a) and stirring until the concentration of lercanidipine hydrochloride dissolved in the crystallization solvent is ≤ 2%; and
c) recovering the solid obtained from step b), and drying said solid to produce the lercanidipine hydrochloride crystalline Form (I).

3. The method according to claim 2, wherein step c) comprises filtering the solid obtained from step b), washing the solid with isopropanol.

4. The method according to anyone of claims 2 or 3, wherein said step a) further comprises filtering the heated solution.

5. The method according to any one of claims 2-4 wherein said step b) comprises cooling the solution to a temperature between 30 and 40°C.

6. The method according to claim 2-5, wherein said step b) further comprises stirring for a period of time of 12-48 hours.

7. The method according to any one of claims 2-6, wherein said drying in step c) takes place in an oven.

## Patentansprüche

1. Lercanidipin-Hydrochlorid der Kristallform (I) mit einem Röntgen-Pulverdiffraktogramm, wie in Fig. 11 gezeigt, zur Verwendung in einem Behandlungsverfahren gegen Hypertonie.

2. Verfahren zur Herstellung von Lercanidipin-Hydrochlorid der Kristallform (I), wie in Anspruch 1 definiert, umfassend:
(a) Hinzufügen von Isopropanol, das ein Maximum an Wasser von 5 % (V/V) enthält, zu einer Lercanidipin-Hydrochlorid-Rohform und Erwärmen unter Rückfluss und Rühren, um eine klare Lösung herzustellen;
(b) Kühlen der Lösung aus Schritt (a) und Rühren, bis die Konzentration an im Kristallisations-Lösungsmittel gelöstem Lercanidipin-Hydrochlorid ≤ 2 % ist; und
(c) Gewinnen des in Schritt (b) erhaltenen Feststoffs und Trocknen des Feststoffs, um Lercanidipin-Hydrochlorid der Kristallform (I) herzustellen.

3. Verfahren gemäss Anspruch 2, worin Schritt (c) das Filtrieren des in Schritt (b) erhaltenen Feststoffs und das Waschen des Feststoffs mit Isopropanol umfasst.

4. Verfahren gemäss einem der Ansprüche 2 oder 3, worin Schritt (a) weiterhin das Filtrieren der erwärmten Lösung umfasst.

5. Verfahren gemäss einem der Ansprüche 2 bis 4, worin Schritt (b) das Kühlen der Lösung auf eine Temperatur zwischen 30 und 40°C umfasst.

6. Verfahren gemäss Ansprüchen 2 bis 5, worin Schritt (b) weiterhin das Rühren über eine Zeitspanne von 12 bis 48 Stunden umfasst.

7. Verfahren gemäss einem der Ansprüche 2 bis 6, worin das Trocknen in Schritt (c) in einem Ofen stattfindet.

## Revendications

1. Forme cristalline de chlorhydrate de lercanidipine (I) ayant un diagramme de diffraction de poudre aux rayons X comme montré dans la figure 11, pour une utilisation dans un procédé de traitement contre l'hypertension.

2. Procédé pour produire une Forme cristalline de chlorhydrate de lercanidipine (I), telle que définie dans la revendication 1, qui comprend :
a) ajouter de l'isopropanol contenant un maximum de 5 % d'eau (en volume) à une Forme de chlorhydrate de lercanidipine brut et chauffer sous reflux et sous agitation pour produire une solution claire;
b) refroidir la solution de l'étape a) et agiter jusqu'à ce que la concentration de chlorhydrate de lercanidipine dissoute dans le solvant de cristallisation soit inférieure ou égale à 2 %; et
c) récupérer le solide obtenu dans l'étape b), et sécher ledit solide afin de produire la Forme cristalline de chlorhydrate de lercanidipine (I).

3. Procédé selon la revendication 2, dans lequel l'étape c) comprend un filtrage du solide obtenu à l'étape b), un lavage du solide avec de l'isopropanol.

4. Procédé selon l'une quelconque des revendications 2 ou 3, dans lequel ladite étape a) comprend en plus un filtrage de la solution chauffée.

5. Procédé selon l'une quelconque des revendications 2 à 4, dans lequel ladite étape b) comprend le refroidissement de la solution à une température entre 30 et 40 °C.

6. Procédé selon les revendications 2 à 5, dans lequel ladite étape b) comprend en outre une agitation pendant une période de temps de 12 à 48 heures.

7. Procédé selon l'une quelconque des revendications 2 à 6, dans lequel ledit séchage dans l'étape c) a lieu dans un four.
